(19) Europäisches Patentamt
European Patent Office
Office européen des brevets

(11) Veröffentlichungsnummer: 0 187 231
B1

(12) # EUROPÄISCHE PATENTSCHRIFT

(45) Veröffentlichungstag der Patentschrifft: **17.01.90**

(51) Int. Cl.⁴: C 07 D 277/66

(21) Anmeldenummer: **85114564.9**

(22) Anmeldetag: **16.11.85**

(54) **Aminophenylbenzthiazole.**

(30) Priorität: **29.11.84 DE 3443594**

(43) Veröffentlichungstag der Anmeldung: **16.07.86 Patentblatt 86/29**

(45) Bekanntmachung des Hinweises auf die Patenterteilung: **17.01.90 Patentblatt 90/03**

(84) Bennante Vertragsstaaten: **CH DE FR GB LI**

(56) Entgegenhaltungen:
**US-A-2 098 599**
**US-A-2 715 629**
**US-A-2 733 242**
**US-A-3 274 171**
**US-A-3 972 875**

**Die Akte enthält technische Angaben, die nach dem Eingang der Anmeldung eingereicht wurden und die nicht in dieser Patentschrift enthalten sind.**

(73) Patentinhaber: **BAYER AG**
**D-5090 Leverkusen 1 Bayerwerk (DE)**

(72) Erfinder: **Fürstenwerth, Hauke, Dr.**
**Morgengraben 3**
**D-5000 Köln 80 (DE)**
Erfinder: **Schündehütte, Karl Heinz, Prof. Dr.**
**Klief 75**
**D-5090 Leverkusen 3 (DE)**

EP 0 187 231 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

**Description**

Gegenstand der vorliegenden Erfindung sind Benzthiazolderivate der Formel

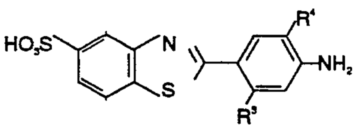

(1)

in welcher

$R^3$, $R^4$ = H, Cl, $CH_3$, $OCH_3$, $OC_2H_5$,

insbesondere solche mit $R^3$, $R^4$ = H sowie

Verfahren zu deren Herstellung, dadurch gekennzeichnet, daß man 4-Nitrobenzoesäurechloride der Formel

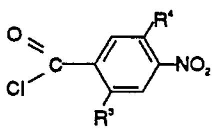

(2)

mit einem 2-Aminothiophenolderivat der Formel

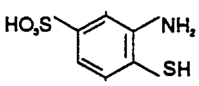

(3)

oder mit Umsetzungsprodukten von 2-Nitrohalogenbenzolen der Formel

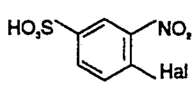

(4)

mit Schwefelalkaliverbindungen, insbes. NaHS, $Na_2S$ oder $K_2S$, umsetzt, die dabei erhaltenen Zwischenprodukte gegebenenfalls ohne Isolierung cyclisiert und zu Verbindungen der Formel (1) reduziert oder umsetzt und die Zwischenprodukte gegebenenfalls ohne Isolierung reduziert und cyclisiert, wobei die Cyclisierung vorzugsweise in wäßrigen Medien bei pH-Werten von 0 bis 5 erfolgt.

Die Reduktionen werden bevorzugt mit sulfidischen Reduktionsmitteln vorgenommen. Die Cyclisierungen werden bevorzugt sauer durchgeführt, können jedoch auch neutral oder alkalisch durchgeführt werden.

Das erfindungsgemäße Verfahren wird im einzelnen so durchgeführt, daß man zunächst eine 4-Nitrobenzoesäure der allgemeinen Formel (5)

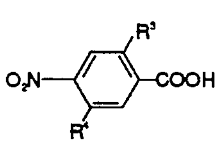

(5)

mit überschüssigem Thionylchlorid oder anderen säurehalogenierenden Verbindungen wie Phosgen, Phosphortrichlorid, Phosphorpentachlorid, Phosphorxychlorid in an sich bekannter Weise (vgl. Houben-Weyl, Methoden der Organischen Chemie, 4. Auflage, Band VIII, Seite 467 (1952); DRP 1 026 750) in die entsprechenden 4-Nitrobenzoesäurechloride der allgemeinen Formel (2)

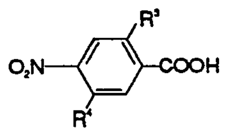

(2)

überführt.

Diese Reaktion wird im allgemeinen in Gegenwart geringer Mengen einer organischen Stickstoffverbindung als Katalysator, vorzugsweise Dimethylformamid oder Pyridin, und bei ansteigender Temperatur, vorzugsweise von 20 bis 100°C, durchgeführt. Die Reaktion der 4-Nitrobenzoesäure (5) zum entsprechenden Nitrobenzoesäurechlorid wird vorzugsweise ohne Lösungsmittel durchgeführt, sie kann jedoch auch in inerten organischen Lösungsmitteln, beispielsweise Methylenchlorid, Chloroform, Tetrachlorkohlenstoff, Dichlorethan, Trichlorethan, n-Hexan, Cyclohexan, Toluol, Xylol, Monochlorbenzol, o-Dichlorbenzol erfolgen.

Die so erhaltene Schmelze oder Lösung des Nitrobenzoesäurechlorids (2) wird - vorzugsweise ohne weitere Reinigung - mit einer wäßrigen Suspension oder Lösung des Aminothiophenols des Formel (3) kondensiert.

Die Kondensationsreaktion wird normalerweise bei pH-Werten zwischen 1 und 10, vorzugsweise 50 bis Temperaturen von -10 bis +100°C, vorzugsweise 50 bis 80°C durchgeführt.

Zur Einhaltung der bei der Kondensationsreaktion optimalen pH-Werte von 1 bis 10, vorzugsweise 5 bis 9, kann man vor oder während der Reaktion zum Abfangen des freiwerdenden Chlorwasserstoffs geeignete Basen, beispielsweise Natriumhydroxid, Natriumcarbonat, Natriumhydrogencarbonat, Natriumacetat, Trinatriumphosphat, Dinatriumphosphat oder Mononatriumphosphat, Natriumformiat, Natriumpropionat zufügen. Bevorzugtes Lösungsmittel ist Wasser.

Das wäßrige Reaktionsmedium kann jedoch auch mit Wasser mischbare Lösungsmittel, beispielsweise Alkohole wie Methanol, Ethanol oder Isopropanol oder mit Wasser nicht mischbare Lösungsmittel, beispielsweise Methylenchlorid, Chloroform, Tetrachlorkohlenstoff, Dichlorethan, Trichlorethan, n-Hexan, Cyclohexan, Toluol, Xylol, Monochlorbenzol, o-Dichlorbenzol enthalten.

Ferner können dem bei den Kondensationsreaktionen verwendeten Reaktionsmedium Tenside, beispielsweise Aniontenside, Kationtenside, Amphotenside onder nicht ionogene Tenside (vgl. Ullmanns Enzyclopädie der technischen Chemie, Band 16, S. 724 - 748 (1965) oder "Surface Activity" von J.L. Moilliet, B. Collie u. Black, 2. Auflage, Kap, 10 - 15) zugesetzt werden.

Der Zusatz von Tensidenerhöht die Reaktionsgeschwindigkeit der Kondensationsreaktionen. Die Kondensationsreaktionen sind normalerweise nach Reaktionszeiten von 1 bis 6 Stunden bei 50°C bis 80°C beendet.

Bei dieser Kondensationsreaktion entstehen zunächst Verbindungen der Formel

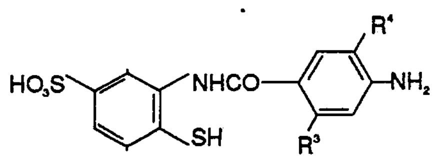

(6)

und/oder

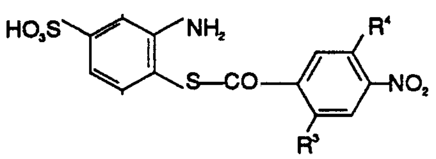

(7)

die zu Benzthiazolen der Formel

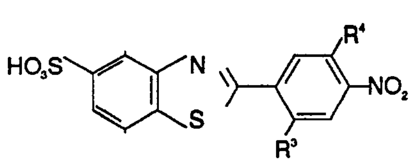

(8)

cyclisiert und weiter zu Benzthiazolen der Formel (1) reduziert werden. Die Cyclisierung von (6) und/oder (7) wird bevorzugt bei pH-Werten von 0 bis 5 durchgeführt.

Nach einer bevorzugten Ausführungsform der vorliegenden Erfindung werden die Reaktionsschritte der Kondensation, Cyclisierung und Reduktion in einem einzigen Verfahrensschritt zusammengefaßt.

Nach dieser bevorzugten Verfahrensvariante (a) werden die Kondensationsprodukte mit sulfidischen Reduktionsmitteln wie z. B. Schwefelwasserstoff, Alkalisulfiden oder Alkalipolysulfiden, insbesondere mit einem Alkalihydrogensulfid, beispielsweise der technisch üblichen Sulfhydratlauge (Natriumhydrogensulfid), reduziert.

Nach einer besonders bevorzugten Ausführungsform der vorliegenden Erfindung werden die Primäraddukte (6) und/oder (7) zunächst zu den Aminoverbindungen der Formel

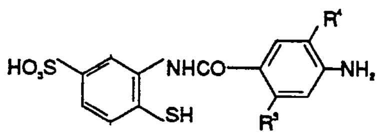

(9)

und/oder

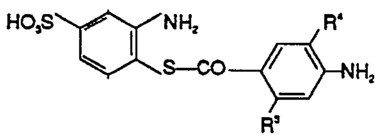

(10)

reduziert, und diese dann bevorzugt bei pH-Werten von 0 bis 5 zu den Benzthiazolderivaten der Formel (1) cyclisiert.

Auch nach dieser Verfahrensvariante können die Kondensationsprodukte der Formel (6)/(7) ohne Isolierung von Zwischenstufen nach einem sogenannten Eintopfverfahren in die Endprodukte der Formel (1) überführt werden.

Hierbei fallen die heterocyclischen Amine der Formel (1) ebenfalls in ausgezeichneter Qualität und in hohen Ausbeuten an, was in Anbetracht des über mehrere Stufen ohne Zwischenisolierung und Reinigung der auftretenden Zwischenstufen verlaufenden Prozesses überraschend ist.

Nach einem ganz besonders bevorzugten Ausführungsverfahren (b) der vorliegenden Erfindung werden Nitrobenzoesäurechloride der Formel (2) mit Umsetzungsprodukten von Nitrohalogenverbindungen der Formel (4) mit Schwefelalkaliverbindungen zur Reaktion gebracht. Bei der Umsetzung von (4) mit Schwefelalkaliverbindungen entstehen je nach Reaktionsbedingungen und Substituenten Aminothiophenole der Formel (3) und/oder Di- und Polysulfide der Formel (11) (vgl. DE-OS-2 127 898, 2 053 715, 2 503 164, J. Mech. Chem. *14*, 248 ff. (1971), Monatshefte f. Chem. *56*, 365 ff. (1930)).

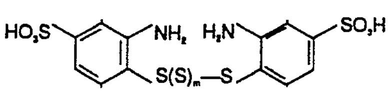

(11)

worin m = 0 bis 10 sein kann, bevorzugt 0 bis 2.

Es ist überraschend, daß sowohl Mischungen aus (3) und (11) als auch (11) allein mit Nitrobenzoylchloriden der Formel (2) und anschließender Reduktion und Cyclisierung, bzw. Cyclisierung und Reduktion, mit hohen Ausbeuten und hoher Reinheit zu Benzthiazolderivaten der Formel (1) reagieren. Nach dieser ganz besonders bevorzugten Verfahrensvariante werden die Reaktionsschritte der Umsetzung mit Schwefelalkali, Umsetzung mit Nitrobenzoylchloriden, Reduktion und Cyclisierung in einem einzigen Verfahrensschritt zusammengefaßt. Für die Reduktion-Cyclisierung bzw. Cyclisierung-Reduktion dieser Verfahrensvariante (b) gelten die gleichen Ausführungen wie in Verfahrensvariante (a). In den Formeln (5) bis (10) haben $R^3$ und $R^4$ die in Formel (1) angegebene Bedeutung.

Nitrobenzoesäuren der Formel (5), Aminothiophenole der Formel (3) und Nitrohalogenbenzole der Formel (4) sind ebenso wie Di- und Polysulfide der Formel (11) bekannt, bzw. lassen sich nach literaturbekannten Verfahren herstellen.

Als 4-Nitrobenzoesäuren (5), die zur Herstellung der zur Kondensationsreaktion benötigten 4-Nitrobenzoesäurechloride (2) geeignet sind, können beispielsweise eingesetzt werden:

4-Nitrobenzoesäure
2-Methyl-4-nitrobenzoesäure
3-Methyl-4-nitrobenzoesäure

2-Methoxy-4-nitrobenzoesäure
3-Methoxy-4-nitrobenzoesäure
2-Methyl-5-methoxy-4-nitrobenzoesäure
3-Ethoxy-4-nitrobenzoesäure
3-Methyl-6-methoxy-4-nitrobenzoesäure
2-Chlor-4-nitrobenzoesäure
3-Chlor-4-nitrobenzoesäure
2-Brom-4-nitrobenzoesäure
3-Brom-4-nitrobenzoesäure
3-Ethoxy-4-nitrobenzoesäure

Als Nitrohalogenbenzole der Formel (4) können Verbindungen eingesetzt werden, in denen Halogen für Fluor, Chlor oder Brom steht.

Die neuen Verbindungen (1) stellen Zwischenprodukte für die Herstellung von Azofarbstoffen dar, sie lassen sich in üblicher Weise diazotieren und mit Kupplungskomponenten umsetzen.

In der US-A-3 274 171 werden Aminophenylbenzthiazole beschrieben, welche sich von den erfindungsgemäßen Aminophenylbenzthiazolen durch die Anwesenheit einer Methylgruppe unterscheiden.

**Beispiel 1**

2-(4'-Aminophenyl)-benzthiazol-5-sulfonsäure

158 g Natriumsalz der 4-Chlor-3-nitrobenzolsulfonsäure (technisch, ca. 83 prozentig) werden in 400 ml Wasser bei ca. 80°C gelöst. Es werden 180 ml 30 prozentiger wäßriger Natriumhydrognsulfidlösung (technisch) in ca. 30 Minuten zugetropft. Es wird eine Stunde bei 90°C nachgerührt. Dann wird auf 70°C abgekühlt und bei dieser Temperatur werden 93 g 4-Nitrobenzoylchlorid eingetragen. Durch Zugabe von Natronlauge wird der pH-Wert des Gemisches bei 8 gehalten. Sobald der pH-Wert konstant bleibt, werden 180 ml 30 prozentiger wäßriger Natriumhydrogensulfidlösung zugegeben und das Reaktionsgemisch eine Stunde am Rückfluß gekocht. Danach wird mit Salzsäure auf pH 1 gestellt und 5 Stunden am Rückfluß gekocht. Nach Abkühlen wird das ausgefallene Produkt isoliert. Das Rohprodukt wird in einer Mischung aus 300 ml Wasser und 150 ml 20 prozentigem Ammoniakwasser auf 90°C erwärmt. Es wird heiß filtriert und das Filtrat noch in der Hitze mit 50 g Kochsalz versetzt. Beim Abkühlen kristallisieren 125 g reines Produkt der Formel

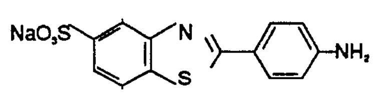

NMR (DMSO) δ = 3,40 (s, 2H), 6,70 (d, 2H),
7,65 (d, 1H), 7,78 (d, 2H),
7,95 (d, 1H), 8,12 (s, 1H)

**Beispiel 2**

158 g 4-Chlor-3-nitrobenzolsulfonsäure-natrium werden wie im Beispiel 1 beschrieben mit NaHS-Lösung umgesetzt. Bei 70°C wird eine Lösung von 93 g 4-Nitrobenzoylchlorid in 130 ml Toluol zugegeben. Mit Natronlauge wird pH 8 gehalten. Sobald der pH-Wert des Gemisches konstant bleibt, werden 180 ml 30 prozentiger wäßriger Natriumhydrogensulfidlösung zugegeben. Danach heizt man das Reaktionsgemisch auf 100 bis 105°C, wobei das eingesetzte Toluol durch Destillation zurückgewonnen wird. Nachdem das Toluol abdestilliert ist, wird mit Salzsäure auf pH 1 gestellt und weitere 3 Stunden am Rückfluß gekocht. Isolierung und Abtrennung von Schwefel werden wie in Beispiel 1 durchgeführt. Man erhält 120 g Produkt, das mit dem aus Beispiel 1 identisch ist.

**Beispiel 3**

158 g 4-Chlor-3-nitrobenzolsulfonsäure-natriumsalz (technisch, ca. 83 prozentig) werden von 400 ml Wasser bei 80°C gelöst. Es wird eine Lösung von 130 g $Na_2S$ x $3H_2O$ und 16 g Schwefel in 100 ml Wasser so zugetropft, daß die Reaktionsmischung leicht am Rückfluß kocht. Nach Zugabe wird noch 2 Stunden am Rückfluß gekocht. Danach wird wie im Beispiel 1 beschrieben mit 83 g 4-Nitrobenzoylchlorid bei 70°C und pH 8 umgesetzt, mit 180 ml 30 prozentiger NaHS-Lösung reduziert und sauer cyclisiert. Isolierung und Reinigung erfolgen wie im Beispiel 1 beschrieben. Man erhält 90 g Produkt, das mit dem aus Beispiel 1 identisch ist.

**Beispiel 4**

103 g 2-Amino-thiophenol-4-sulfonsäure werden in 400 ml Wasser bei 70°C mit 93 g 4-Nitrobenzoylchlorid versetzt. Durch Zugabe von Natronlauge wird pH 8 gehalten. Sobald der pH-Wert des Reaktionsgemisches konstant bleibt, werden wie in Beispiel 1 180 ml 30 prozentige NaHS-Lösung zugegeben, anschließend sauer cyclisiert und wie in Beispiel 1 aufgearbeitet. Man erhält 135 g Produkt, das mit dem aus Beispiel 1 identisch ist.

**Beispiel 5**

158 g 4-Chlor-3-nitrobenzolsulfonsäure-natriumsalz werden wie in Beispiel 1 beschrieben mit NaHS-Lösung und 4-Nitrobenzoylchlorid bei pH 7,5 bis 8,5 umgesetzt. Sobald der pH-Wert des Reaktionsgemisches konstant bleibt, wird mit HCl auf pH 1 gestellt und eine Stunde am Rückfluß gekocht. Nach Abkühlen wird isoliert und mit $H_2O$ gewaschen. Die feuchte Paste des Produktes mit der Formel

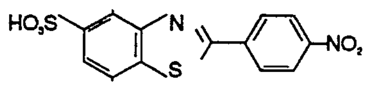

wird in 500 ml Wasser verrührt und bei 80°C beginnend mit 180 ml 30 prozentiger NaHS-Lösung versetzt. Es wird 1 Stunde am Rückfluß gekocht, mit Salzsäure auf pH 1 gestellt und isoliert. Das Rohprodukt wird in einer Lösung von 20 g NaOH in 500 ml Wasser auf 90°C erwärmt, filtriert, das Filtrat mit HCl auf pH 1 gestellt, kalt gerührt, isoliert, geaschen und getrocknet.

Man erhält 120 g Produkt der Formel

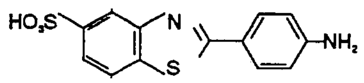

Arbeitet man nach den Angaben der Beispiele 1 bis 6 und verwendet anstelle von 4-Nitrobenzoylchlorid die Säurechloride der in Tabelle 1 dieser Anmeldung aufgezählten Nitrobenzoesäuren, so erhält man entsprechend substituierte Benzthiazol-5-sulfonsäurederivate.

**Beispiele 6 - 8**

Analog Beispiel 1 werden hergestellt:

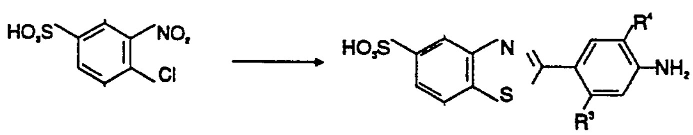

| Beispiel | $R^3$ | $R^4$ |
|---|---|---|
| 6 | H | $CH_3$ |
| 7 | H | $OCH_3$ |
| 8 | $OCH_3$ | $OCH_3$ |

**Patentansprüche**

1. Benzthiazolderivate der Formel

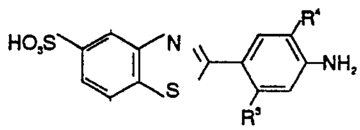

(1)

in welcher

$R^3$, $R^4$ = H, Cl, $CH_3$, $OCH_3$, $OC_2H_5$.

2. Benzthiazolderivate des Anspruchs 1 mit $R^3$, $R^4$ = H.

3. Verfahren zur Herstellung der Benzthiazolderivate des Anspruchs 1, dadurch gekennzeichnet, daß man 4-Nitrobenzoesäurechloride der Formel

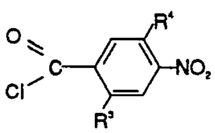

(2)

mit 2-Aminothiophenolderivaten der Formel

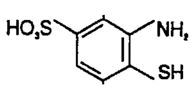

(3)

oder mit Umsetzungsprodukten von 2-Nitrohalogenbenzolen der Formel

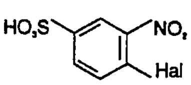

(4)

mit Schwefelalkaliverbindungen umsetzt, die dabei erhaltenen Zwischenprodukte gegebenenfalls ohne Isolierung cyclisiert und zu Verbindungen der Formel (1) reduziert oder umsetzt und die Zwischenprodukte gegebenenfalls ohne Isolierung reduziert und cyclisiert.

4. Verfahren gemäß Anspruch 3, dadurch gekennzeichnet, daß die Cyclisierung in wäßrigen medien bei pH-Werten von 0 bis 5 erfolgt.

**Claims**

1. Benzothiazole derivatives of the formula

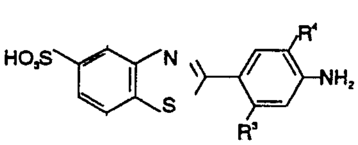

(1)

in which

$R^3$, $R^4$ = H, Cl, $CH^3$, $OCH_3$, $OC_2H_5$.

2. Benzothiazole derivatives of claim 1 where $R^3$, $R^4$ = H.

3. Process for preparing the benzothiazole derivatives of claim 1, characterised in that 4-nitrobenzoyl chlorides of the formula

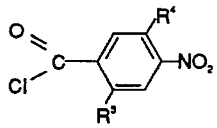

(2)

are reacted with 2-aminothiophenol derivatives of the formula

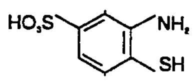

(3)

or with reaction products of 2-nitrohalobenzenes of the formula

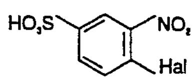

(4)

with sulphur alkali metal compounds, the resulting intermediate products - with or without isolation - are cyclised and reduced or converted to compounds of the formula (1) and the intermediate products - with or without isolation - are reduced and cyclised.

4. Process according to claim 3, characterised in that the cyclisation is carried out at pH 0 - 5 in aqueous media.

**Revendications**

1. Dérivés du benzothiazole de formule

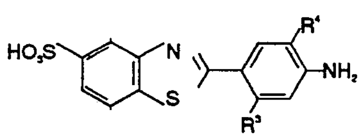

(1)

dans laquelle

$R^3$, $R^4$ = H, Cl, $CH_3$, $OCH_3$, $OC_2H_5$.

2. Dérivés du benzothiazole de la revendication 1, dans lesquels $R^3$, $R^4$ = H.

3. Procédé de préparation des dérivés du benzothiazole de la revendication 1, caractérisé en ce que l'on fait réagir des chlorures d'acides 4-nitrobenzoïques de formule

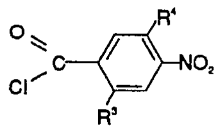

(2)

avec des dérivés du 2-aminothiophénol de formule

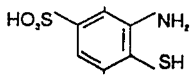

(3)

ou avec des produits de réaction de 2-nitrohalogénobenzènes de formule

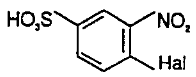

(4)

et de composés du soufre et de métaux alcalins, on cyclise les produits intermédiaires ainsi obtenus, le cas échéant sans les isoler, et on les réduit ou on les convertit en composés de formule (1) et on réduit et cyclise les produits intermédiaires éventuellement sans tes isoler.

4. Procédé selon la revendication 3, caractérisé en ce que la cyclisation est effectuée en milieu aqueux à des pH de 0 à 5.